(19) Europäisches Patentamt / European Patent Office / Office européen des brevets

(11) **EP 3 366 372 A1**

(12) **EUROPEAN PATENT APPLICATION**
published in accordance with Art. 153(4) EPC

(43) Date of publication:
**29.08.2018 Bulletin 2018/35**

(51) Int Cl.:
***B01J 23/887*** *(2006.01)*     ***B01J 35/08*** *(2006.01)*
***B01J 37/08*** *(2006.01)*     ***C07B 61/00*** *(2006.01)*
***C07C 5/48*** *(2006.01)*     ***C07C 11/167*** *(2006.01)*

(21) Application number: **16857433.3**

(22) Date of filing: **18.10.2016**

(86) International application number:
**PCT/JP2016/080838**

(87) International publication number:
**WO 2017/069119 (27.04.2017 Gazette 2017/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **19.10.2015 JP 2015205388**

(71) Applicant: **Nippon Kayaku Kabushiki Kaisha Tokyo 100-0005 (JP)**

(72) Inventors:
• **MOTOMURA Hiroki**
  **Sanyoonoda-shi**
  **Yamaguchi 757-0002 (JP)**
• **NAKAZAWA Yuta**
  **Sanyoonoda-shi**
  **Yamaguchi 757-0002 (JP)**
• **OKUMURA Shigeki**
  **Sanyoonoda-shi**
  **Yamaguchi 757-0002 (JP)**

(74) Representative: **Gille Hrabal**
  **Brucknerstrasse 20**
  **40593 Düsseldorf (DE)**

(54) **CATALYST FOR CONJUGATED DIOLEFIN PRODUCTION, AND METHOD FOR PRODUCING SAME**

(57)    Provided are a catalyst that may suppress the production of a coke-like material and may improve the long-term stability of the reaction, in a reaction for producing a conjugated diolefin from a mixed gas including a monoolefin having 4 or more carbon atoms and molecular oxygen by catalytic oxidative dehydrogenation; and a method for producing the catalyst.

Provided is a composite metal oxide catalyst for conjugated diolefin production, used for producing a conjugated diolefin from a mixed gas including a monoolefin having 4 or more carbon atoms and molecular oxygen by a catalytic oxidative dehydrogenation reaction, the catalyst having a relative intensity ratio of X-ray diffraction peaks represented by the following Formula (A):

$$0.9 < Pr < 3.0 \qquad (A)$$

$$Pr = Pi1/Pi2$$

(in the formula, Pi1 represents the maximum peak height at a $2\theta$ value in the range of $26.4° \pm 0.3°$ in the X-ray diffraction peaks; Pi2 represents the maximum peak height at a $2\theta$ value in the range of $28.5° \pm 0.3°$ in the X-ray diffraction peaks; and Pr represents the relative intensity ratio of Pi1 with respect to Pi2).

EP 3 366 372 A1

[Figure 1]

**Description**

Technical Field

The present invention relates to a catalyst for producing a conjugated diolefin from a mixed gas including a monoolefin having 4 or more carbon atoms and molecular oxygen by a catalytic oxidative dehydrogenation reaction, and a method for producing the catalyst.

Background Art

Butadiene, which is a raw material of synthetic rubbers or the like, has been conventionally produced industrially by thermal decomposition of a naphtha fraction and extraction; however, since there are concerns about deterioration of stable supply to the market in the future, there is a demand for a new method for producing butadiene. Thus, attention has been paid to a method of oxidatively dehydrogenating n-butene from a mixed gas including n-butene and molecular oxygen in the presence of a catalyst. However, there are concerns that as a coke-like material originating from an intended product and/or reaction by-products is precipitated in or adheres to the catalyst surface, inert materials, the interior of a reaction tube, or the interior of the subsequent process facilities, various problems may be brought about, such as inhibition of the circulation of the reaction gas in an industrial plant, blocking of the reaction tube, and shutdown of the plant or a decrease in the yield resulting therefrom. For the purpose of avoiding the problems described above, in an industrial plant, generally, the reaction is stopped before blocking occurs, and a regeneration treatment of combusting and removing the coke-like material by means of temperature increase of the reaction bath temperature or the like is carried out. However, since stopping a reaction that is in steady operation and then performing a regeneration treatment leads to deterioration of economic efficiency in an industrial plant, it is desirable that production of the coke-like material is suppressed as far as possible. Thus, it is known to make improvements in the catalyst composition, reaction conditions, and the like as described below, for the purpose of suppressing the generation of coke-like materials.

Regarding those patent literatures related to the catalyst composition, Patent Literature 1 discloses a catalyst composition that suppresses side-production of a coke-like material and enables stable continuation of the reaction, and Patent Literature 3 discloses a catalyst composition and the acidity of the catalyst that give high yield of a conjugated diolefin. In Patent Literature 4, the X-ray diffraction peak ratio of a catalyst that may suppress the generation of a coke-like material is defined. Furthermore, regarding non-patent literatures related to catalysts, an interaction between different crystal structures in a catalyst for unsaturated aldehyde production has been reported in Non-Patent Literature 1, and the crystal structure of a catalyst for conjugated diolefin production showing a high conversion ratio has been reported in Non-Patent Literature 2. Furthermore, regarding patent literatures related to the reaction conditions, reaction conditions that prevent accumulation of a coke-like material on a catalyst are defined in Patent Literature 2. In regard to all of these patent literatures, when the economic efficiency in an industrial plant, that is, the amount of precipitation of the coke-like material with respect to the duration of the reaction, the frequency of regeneration, and the like are taken into consideration, it is inappropriate to say that the production of coke-like materials has been sufficiently suppressed. Thus, proposal of a catalyst composition that may suppress the production of a coke-like material and may improve the long-term stability of the reaction, and the reaction conditions for the catalyst, is desirable.

Furthermore, from the viewpoint of economic efficiency in industrial plants, it is definitely important that butadiene as a target product is obtained with high yield. A low butadiene yield means that the yield of reaction by-products is relatively high; however, in this case, in order to obtain butadiene of high purity as the final manufactured product in an industrial plant, purification systems with superior performance are needed, and it is concerned that the equipment cost of these systems increases.

Furthermore, from the viewpoint of the long-term stability of the reaction in an industrial plant, it is also important that the mechanical strength of the catalyst itself is high. When the catalyst has insufficient mechanical strength, problems such as damage of the catalyst caused by the production of a coke-like material, and/or damage of the catalyst caused by a combustion gas in a regeneration treatment, and/or deterioration of the catalyst may be considered. Furthermore, it is concerned that the damaged catalyst accumulates inside the reactor, and this leads to problems such as an increase in the pressure loss, undesired reactions caused by the catalyst locally accumulated inside the reactor, and incorporation of the catalyst into the purification systems in the subsequent stages.

Citation List

Patent Literature

Patent Literature 1: JP 2013-100244 A
Patent Literature 2: JP 2011-148765 A
Patent Literature 3: JP 2013-146655 A
Patent Literature 4: JP 2013-202459 A

Non Patent Literature

Non-Patent Literature 1: S.R.G. Carrazfin, et al. (1996) App. Cat. A., 135, 95-123
Non-Patent Literature 2: J.C. Jung, et al. (2008) Cat. Com., 9, 2059-2062

Summary of Invention

Technical Problem

An object of the present invention is to provide a catalyst that may suppress the production of a coke-like material and may improve the long-term stability of the reaction, in a reaction for producing a conjugated diolefin from a mixed gas including a monoolefin having 4 or more carbon atoms and molecular oxygen by catalytic oxidative dehydrogenation, and to provide a method for producing the catalyst.

Solution to Problem

The inventors of the present invention conducted a thorough investigation in order to solve the problems described above, and as a result, the inventors found that when the relative intensity ratio of a particular crystal phase in relation to the X-ray diffraction peaks of a catalytically active component satisfies a particular range, the production of a coke-like material may be significantly suppressed, and the problems described above may be solved. Thus, the inventors completed the present invention.

The present invention has the features of the following items (1) to (7) singly or in combination. That is, the present invention relates to:

(1) a composite metal oxide catalyst for conjugated diolefin production, used for producing a conjugated diolefin from a mixed gas including a monoolefin having 4 or more carbon atoms and molecular oxygen by a catalytic oxidative dehydrogenation reaction, the catalyst having a relative intensity ratio of X-ray diffraction peaks represented by the following Formula (A):

$$0.9 < Pr < 3.0 \qquad (A)$$

$$Pr = Pi1/Pi2$$

(in the formula, Pi1 represents the maximum peak height at a $2\theta$ value in the range of $26.4° \pm 0.3°$ in the X-ray diffraction peaks; Pi2 represents the maximum peak height at a $2\theta$ value in the range of $28.5° \pm 0.3°$ in the X-ray diffraction peaks; and Pr represents the relative intensity ratio of Pi1 with respect to Pi2);
(2) the composite metal oxide catalyst for conjugated diolefin production according to (1), wherein the composite metal oxide satisfies the following compositional formula (D) :

$$Mo_{12}Bi_aFe_bCo_cNi_dX_eY_fZ_gO_h \dots \qquad (D)$$

(in the formula, X represents at least one element of alkali metal selected from lithium, sodium, potassium, rubidium, and cesium; Y represents at least one element of alkaline earth metal selected from magnesium, calcium, strontium, and barium; Z represents at least one element selected from lanthanum, cerium, praseodymium, neodymium, samarium, europium, antimony, tungsten, lead, zinc, and thallium; a, b, c, d, e, f, and g respectively represent the atomic ratio of each component with respect to molybdenum 12, and are in the following ranges: $0.2 \leq a \leq 2.0$, $0.6 < b < 3.4$, $5.0 < c < 8.0$, $0 < d < 3.0$, $0 < e < 0.5$, $0 \leq f \leq 4.0$, and $0 \leq g \leq 2.0$; and h represents a value satisfying the oxidation state of the other elements);

(3) a supported catalyst for conjugated diolefin production, the supported catalyst including the composite metal oxide catalyst according to (1) or (2) supported on a support;

(4) the supported catalyst for conjugated diolefin production according to (3), wherein the average particle size is from 3.0 mm to 10.0 mm, and the support ratio of the composite metal oxide catalyst is from 20% by weight to 80 by weight;

(5) a method for producing the catalyst for conjugated diolefin production according to any one of (1) to (4), the method including:

preparing a solution or a slurry including compounds containing the various metals of the composite metal oxide;

drying the solution or slurry to obtain a dried powder;

preliminarily calcining the dried powder at a temperature of from 200°C to 600°C;

molding the preliminarily calcined powder; and

subjecting the molded article to main calcination at a temperature of from 300°C to 600°C;

(6) the method for producing the catalyst for conjugated diolefin production according to (5), wherein the solution or slurry is a solution or slurry including a compound containing molybdenum; a compound containing bismuth; a compound containing iron; a compound containing cobalt; a compound containing nickel; a compound containing at least one element of alkali metal selected from lithium, sodium, potassium, rubidium, and cesium; a compound containing at least one element of alkaline earth metal selected from magnesium, calcium, strontium, and barium; and a compound containing at least one element Z selected from lanthanum, cerium, praseodymium, neodymium, samarium, europium, antimony, tungsten, lead, zinc, and thallium; and

(7) a method for producing the supported catalyst for conjugated diolefin production according to (3) or (4), wherein the molding is coating a support with the composite metal oxide together with a binder.

Advantageous Effects of Invention

The present invention provides a catalyst that may suppress the production of a coke-like material and may improve the long-term stability of the reaction, in a reaction for producing a conjugated diolefin from a mixed gas including a monoolefin having 4 or more carbon atoms and molecular oxygen by catalytic oxidative dehydrogenation, and also provides a method for producing the catalyst.

Brief Description of Drawings

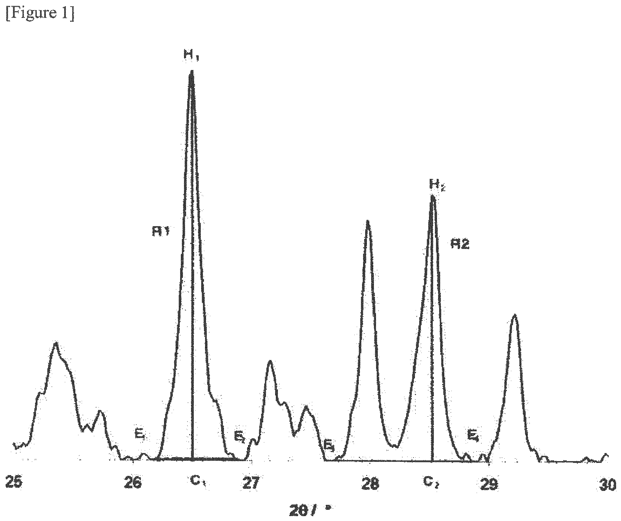

Fig. 1 is a diagram showing the results of an analysis of X-ray diffraction peaks according to Example 1.

Description of Embodiments

The catalyst of the present invention may be used in a reaction for producing a conjugated diolefin from a mixed gas including a monoolefin having 4 or more carbon atoms and molecular oxygen by a catalytic oxidative dehydrogenation reaction. Preferably, the catalyst of the present invention may be used in a reaction for producing butadiene from a mixed gas including n-butene and molecular oxygen by a catalytic oxidative dehydrogenation reaction.

n-Butene according to the present invention means a gas of a single component, or a mixed gas including at least one component, from among 1-butene, trans-2-butene, cis-2-butene, and isobutylene. More strictly, butadiene is intended to mean 1,3-butadiene.

In the following description, the method for analyzing X-ray diffraction peaks according to the present invention will be explained. The term X-ray diffraction peaks refers to a chart of scattering intensities for particular values of the X-ray diffraction angle $2\theta$, and in regard to the analyzer, analysis conditions and the like for the X-ray diffraction peaks, the details do not matter as long as these matters are generally known.

Next, a method for calculating the relative intensity ratio Pr of X-ray diffraction peaks according to the present invention will be described.

For convenience, with regard to catalyst 1 of Example 1 that will be described below, definitions will be explained using an X-ray diffraction chart (Fig. 1) obtained as a result of measurement. Peak intensities Pi1 and Pi2 in Fig. 1 are the lengths of line segments $C_1H_1$ and $C_2H_2$, respectively. $H_1$ and $H_2$ are the apices of peaks at $2\theta$ values of $26.4° \pm 0.3°$ and $28.5° \pm 0.3°$, respectively. $E_1$, $E_2$, $E_3$, and $E_4$ are points at which the X-ray diffraction peaks have the minimum values at $2\theta$ values in the range of $26.1° \pm 0.3°$, in the range of $26.8° \pm 0.3°$, in the range of $27.5° \pm 0.3$, and $28.8° \pm 0.3°$. $C_1$ is the intersection point of a perpendicular line dropped from $H_1$ to the $2\theta$ axis and line segment $E_1E_2$, and $C_2$ is the intersection point of a perpendicular line dropped from $H_2$ to the $2\theta$ axis and line segment $E_3E_4$. The relative intensity ratio Pr is calculated by the following formula from Pi1 and Pi2 thus obtained.

```
Pr = Pi1/Pi2
```

The catalyst of the present invention is a composite metal oxide catalyst in which the relative intensity ratio Pr of X-ray diffraction is represented by the following formula (A).

$$0.9 < Pr < 3.0 \qquad (A)$$

```
Pr = Pi1/Pi2
```

According to the present invention, the details on how a composite metal oxide catalyst that satisfies the above-described formula (A) is capable of suppressing the production of the coke-like material in a reaction for producing a conjugated diolefin from a mixed gas including a monoolefin having 4 or more carbon atoms and molecular oxygen, are not clearly understood. However, it is disclosed in Non-Patent Literature 1 that in a case in which bismuth molybdate and cobalt molybdate co-exist in a catalyst for the production of methacrolein from isobutylene, the production of a coke-like material is suppressed compared to the case in which simple bismuth molybdate exists alone. Furthermore, it is known that cobalt molybdate may adopt two kinds of crystal structures such as $\alpha$-CoMoO$_4$ and $\beta$-CoMoO$_4$, and it is known from Non-Patent Literature 2 that when the existence ratio of $\alpha$-CoMoO$_4$ and $\beta$-CoMoO$_4$ in a catalyst for the production of butadiene from n-butene is changed, the activity of the catalyst is changed. Thus, it is considered that the existence ratio of $\alpha$-CoMoO$_4$ and $\beta$-CoMoO$_4$ contributes to the various chemical properties of the catalyst. From these, it is speculated that in a case in which bismuth molybdate and $\alpha$-CoMoO$_4$ as well as $\beta$-CoMoO$_4$ exist at a particular ratio range, an interaction occurs between bismuth molybdate and cobalt molybdate, and the amount of carbon precipitation is reduced.

Regarding the means for adjusting the relative intensity ratio Pr to be higher than 0.9 and lower than 3.0, it may be considered to regulate the composition of the catalytically active components, and/or to regulate the calcination conditions employed during catalyst production.

The composition of catalytically active components may refer to, for example, the atomic ratio of other elements such as Bi, Fe, and Co with respect to Mo, and above all, with regard to the amount of Bi, when the amount of Bi increases, the amount of Pr tends to decrease, while when the amount of Bi decreases, the amount of Pr tends to increase. When the atomic ratio of Mo is adjusted to 12 at the time of producing the catalyst, the atomic ratio of Bi is preferably from 0.2 to 2.0, and more preferably from 0.3 to 1.5. Furthermore, regarding the regulation of the calcination conditions, the calcination temperature, the time for temperature increase, the calcination time, and the like may be regulated. Here, the calcination temperature means the target temperature in a calcination process, the time for temperature increase means the time taken to reach the calcination temperature, and the calcination time means the time for maintaining the calcination temperature. Regarding the adjustments of the calcination conditions, in the main calcination, which is a final heat treatment process carried out during catalyst production, the time for temperature increase is usually in the range of from 2 hours to 20 hours, preferably from 3 hours to 15 hours, and even more preferably from 4 hours to 10 hours. As the time for temperature increase becomes longer, the cost required for the production of the catalyst tends to increase, and as the time for temperature increase becomes shorter, Pr tends to decrease. A preferred range of the calcination temperature for the main calcination is from 300°C to 600°C, and more preferably from 450°C to 600°C.

The catalyst of the present invention preferably has a composition represented by the following formula (D).

$$Mo_{12}Bi_aFe_bCo_cNi_dX_eY_fZ_gO_h \ldots \qquad (D)$$

(In the formula, X represents at least one element of alkali metal selected from lithium, sodium, potassium, rubidium, and cesium; Y represents at least one element of alkaline earth metal selected from magnesium, calcium, strontium, and barium; Z represents at least one element selected from lanthanum, cerium, praseodymium, neodymium, samarium, europium, antimony, tungsten, lead, zinc, and thallium; a, b, c, d, e, f, and g respectively represent the atomic ratio of each component with respect to molybdenum 12, and are in the following ranges: $0.2 \leq a \leq 2.0$, $0.6 < b < 3.4$, $5.0 < c < 8.0$, $0 < d < 3.0$, $0 < e < 0.5$, $0 \leq f \leq 4.0$, and $0 \leq g \leq 2.0$; and h represents a value that satisfies the oxidation state of the other elements.)

There are no particular limitations on the raw materials of the various metal elements for obtaining the catalyst of the present invention; however, nitrates, nitrites, sulfates, ammonium salts, organic acid salts, acetates, carbonates, subcarbonates, chlorides, inorganic acids, inorganic acid salts, heteropolyacids, heteropolyacid salts, hydroxides, oxides, metals, alloys, and the like, all of which include at least one of the various metal elements, and mixtures thereof may be used. Specific examples thereof include the following compounds. Regarding the supply source of Mo, ammonium molybdate is preferred. In particular, ammonium molybdate includes a plurality of kinds of compounds, such as ammonium dimolybdate, ammonium tetramolybdate, and ammonium heptamolybdate; however, among them, ammonium heptamolybdate is most preferred. Regarding the raw material of bismuth component, bismuth nitrate is preferred. Regarding the raw materials of iron, cobalt, nickel, and other elements, usually, oxides, or nitrates, carbonates, organic acid salts, hydroxides and the like, which may be converted to oxides by firing, as well as mixtures thereof may be used.

The method for producing the catalyst of the present invention is not particularly limited; however, the production methods may be roughly classified into the following two types of production methods. For convenience, these methods will be referred to as Method (A) and Method (B) in the present invention. Method (A) is a method of obtaining a composite metal oxide as a powder and then molding this powder, and Method (B) is a method of bringing a solution or slurry that includes compounds containing various metals of a composite metal oxide, into contact with a support that has been molded in advance, and thereby supporting the compounds on the support. The details of Method (A) and Method (B) will be described below.

A catalyst production method according to Method (A) will be described below. The order of the various processes is described below as a preferred example; however, it should be noted that there are no limitations on the sequence of the various processes, the number of processes, and combinations of the various processes for obtaining a final manufactured catalyst product.

Step (A1) Compounding and drying

A solution or slurry that includes compounds containing the various metals of a composite metal oxide is prepared, and the solution or slurry is subjected to the processes of a precipitation method, a gelation method, a co-precipitation method, a hydrothermal synthesis method, or the like. Subsequently, a dried powder of the present invention is obtained by using a known drying method such as a dry spraying method, an evaporation drying method, a drum drying method, or a freeze-drying method. For this mixed solution or slurry, the solvent may be any of water, an organic solvent, or a mixed solution thereof, and there are also no limitations on the raw material concentrations of the active components of the catalyst. Furthermore, there are no particular limitations on compounding conditions and drying conditions, such as the liquid temperature of this mixed solution or slurry and the atmosphere; however, it is definitely adequate to select the conditions from appropriate ranges by taking into consideration of the performance, mechanical strength, moldability, production efficiency, and the like of the catalyst finally obtained. Among these, the most preferred method for the present invention is a method of forming a mixed solution or slurry of the raw materials of the active components of a catalyst under the conditions of from 20°C to 90°C, introducing this into a spray dryer, and regulating the hot air inlet temperature, the pressure inside the spray dryer, and the flow rate of the slurry such that the dryer outlet temperature would be from 70°C to 150°C, and the average particle size of the dried powder thus obtained would be from 10 $\mu$m to 700 $\mu$m.

Step (A2) Preliminary calcination

The dried powder thus obtained is preliminarily calcined at a calcination temperature of from 200°C to 600°C, and thus the composite metal oxide (preliminarily calcined powder) of the present invention may be obtained. In the present invention, a composite metal oxide may be referred to as a preliminarily calcined powder. With regard to the conditions for the preliminary calcination as well, there are no particular limitations on the calcination time or the atmosphere employed during calcination, and the technique of calcination is also not particularly limited and may be selected from a fluidized bed, a rotary kiln, a muffle furnace, a tunnel calcination furnace, and the like. It is definitely adequate to select appropriate ranges of the conditions by taking into consideration of the performance, mechanical strength, moldability, production efficiency, and the like of the catalyst finally obtained. Among these, a method of performing preliminary calcination in a tunnel calcination furnace at a calcination temperature of from 300°C to 600°C for a calcination time of from 1 hour to 12 hours in an air atmosphere is preferable for the present invention.

Step (A3) Molding

The preliminarily calcined powder obtained as described above may be directly used as a catalyst; however, the preliminarily calcined powder may also be used after molding. The shape of the molded article is not particularly limited and may be selected from a spherical shape, a cylindrical shape, a ring shape, and the like. However, the shape should be selected by taking into consideration of the mechanical strength of the catalyst that is finally obtainable after a series of production processes, the reactor, the production efficiency for the production, and the like. There are also no particular limitations on the molding method; however, when the support, molding aids, strength enhancing agent, binder, and the like that will be described in the following paragraph are added to the preliminarily calcined powder and molded into a cylindrical shape or a ring shape, a molded article is obtained by using a tablet molding machine, an extrusion molding machine, or the like. When the mixture is molded into a spherical shape, a molded article is obtained by using a granulator or the like. Among these, a method of supporting and molding the preliminarily calcined powder on an inert spherical support by coating the inert spherical support with the preliminarily calcined powder by a tumbling granulation method is preferred for the present invention.

Regarding the material for the support, known materials such as alumina, silica, titanium, zirconia, niobia, silica-alumina, silicon carbide, a carbide, and mixtures thereof may be used. Furthermore, there are no particular limitations on the particle size, the water absorption rate, and mechanical strength of the support, the degrees of crystallization of various crystal phases, the mixing ratio of the crystal phases, and the like, and it is definitely adequate to select appropriate ranges of the conditions by taking into consideration of the performance, moldability, production efficiency, and the like of the catalyst finally obtained. The mixing ratio of the support and the preliminarily calcined powder is calculated as a support ratio by the following formula, based on the feed weights of the various raw materials.

```
Support ratio (wt%) = (Weight of preliminarily

calcined powder used for molding)/{(weight of preliminarily

calcined powder used for molding) + (weight of support used

for molding)} × 100
```

Regarding the raw materials other than the preliminarily calcined powder used for molding, molding may be carried out by using the preliminarily calcined powder, as well as any types of a molding aid such as crystalline cellulose; a strength enhancing agent such as ceramic whiskers; a binder such as an alcohol, a diol or a triol; an aqueous solution thereof; and the like at any arbitrary mixing proportions, and there are no particular limitations. Furthermore, elements can also be introduced onto the outermost surface of the catalyst in an embodiment that is different from Step (A1), by using the solution of catalyst raw materials for this binder.

Step (A4) Main calcination

It is preferable that the preliminarily calcined powder or the molded article obtained as described above is calcined again (main calcination) at a calcination temperature of from 300°C to 600°C before being used in a reaction. Also, in connection with the main calcination, there are no particular limitations on the calcination time or the atmosphere employed during calcination, and the technique for calcination is also not particularly limited and may be selected from a fluidized bed, a rotary kiln, a muffle furnace, a tunnel calcination furnace, and the like. It is definitely adequate to select appropriate ranges of the conditions by taking into consideration of the performance and mechanical strength of the catalyst finally obtained, the production efficiency, and the like. Among these, the most preferred method for the present invention is a method of performing calcination in a tunnel calcination furnace at a calcination temperature of from 450°C to 600°C for a calcination time of from 1 hour to 12 hours in an air atmosphere. At this time, the time for temperature increase is usually from 2 hours to 20 hours, and the calcination may be carried out for a time in the range of preferably from 3 hours to 15 hours, and more preferably from 4 hours to 10 hours.

Next, a catalyst production method according to Method (B) will be described below. Various processes will be sequentially described in the following description; however, there are no particular limitations on the sequence of the various processes, the number of processes, and combinations of the various processes for obtaining the final manufactured product of catalyst.

Step (B1) Impregnation

A solution or slurry that includes compounds containing the various metals of a composite metal oxide is prepared, and a molded support or a catalyst obtained by Method (A) is impregnated with this solution or slurry. Thus, a molded article is obtained. Here, the method for supporting the active components of the catalyst by impregnation is not particularly limited and may be selected from a dipping method, an incipient wetness method, an ion exchanging method, a pH swing method, and the like. As the solvent for the solution or the slurry, any of water, an organic solvent, and a mixed solution thereof may be used, and there are no limitations on the raw material concentrations of the active components of the catalyst. Furthermore, there are also no particular limitations on the liquid temperature of the mixed solution or the slurry, the pressure applied to the liquid, and the atmosphere around the liquid. However, it is definitely adequate to select appropriate ranges of the conditions by taking into consideration of the performance, mechanical strength, moldability, production efficiency, and the like of the catalyst finally obtained. The shapes of the molded support and the catalyst obtained by Method (A) are all not particularly limited and may be selected from a spherical shape, a cylindrical shape, a ring shape, a powdered form, and the like, and there are also no particular limitations on the material, particle size, water absorption ratio, and mechanical strength.

Step (B2) Drying

The molded article obtained as described above is subjected to a heat treatment at a temperature in the range of from 20°C to 200°C using a known drying method such as an evaporation drying method, a drum drying method, or a freeze-drying method, and thus the catalyst dry molded product of the present invention is obtained. There are no particular limitations on the drying time or the atmosphere employed upon drying, and the technique for drying is also not particularly limited and may be selected from a fluidized bed, a rotary kiln, a muffle furnace, a tunnel calcination furnace, and the like. It is definitely adequate to select appropriate ranges of these conditions by taking into consideration of the performance, mechanical strength, moldability, production efficiency, and the like of the catalyst finally obtained.

Step (B3) Main calcination

The catalyst dry molded product obtained as described above is subjected to a heat treatment at a calcination temperature of from 300°C to 600°C, and thus the catalyst of the present invention is obtained. Here, there are no particular limitations on the calcination time or the atmosphere employed during calcination, and the technique of calcination is also not particularly limited and may be selected from a fluidized bed, a rotary kiln, a muffle furnace, a tunnel calcination furnace, and the like. It is definitely adequate to select appropriate ranges of these conditions by taking into consideration of the performance, mechanical strength, moldability, production efficiency, and the like of the catalyst finally obtained. Among these, the most preferred method for the present invention is a method of performing calcination in a tunnel calcination furnace at a main calcination temperature of from 450°C to 600°C for a calcination time of from 1 hour to 12 hours in an air atmosphere. At this time, the time for temperature increase is usually from 2 hours to 20 hours, and it is desirable to perform calcination for a time in the range of preferably from 3 hours to 15 hours, and more preferably from 4 hours to 10 hours.

The catalyst obtained by the production as described above is not particularly limited in view of the shape or size of the catalyst; however, when the workability of packing into the reaction tube, the pressure loss in the reaction tube after packing, and the like are considered, the shape is preferably a spherical shape, the average particle size is preferably from 3.0 mm to 10.0 mm, and the support ratio of the catalytically active components is preferably from 20% by weight to 80% by weight.

It is preferable that the catalyst of the present invention has a certain mechanical strength at least before the initiation of the reaction, as will be disclosed below. As described above, in the case of performing a reaction for producing a conjugated diolefin from a monoolefin having 4 or more carbon atoms particularly in an industrial plant, the production of a coke-like material and a regeneration treatment for the combustion of the coke-like material are carried out in many cases. Thus, if the catalyst has insufficient mechanical strength, damage of the catalyst caused by the production of a coke-like material inside the catalyst, and damage of the catalyst and/or deterioration of the catalyst caused by the combustion gas in the regeneration treatment may be considered. Also, it is concerned that the damaged catalyst accumulates inside the reactor, and this leads to various problems such as an increase in the pressure loss, undesirable reactions and/or a decrease in the yield caused by the catalyst that has locally accumulated inside the reactor, and incorporation of the catalyst into the purification systems in the subsequent stages. Here, the mechanical strength of a catalyst is affected by various factors in the production process, such as the types or amounts of the various strength enhancing agents and binders added at the time of molding, or a combination thereof; the atomic ratio of the catalyst composition, the phase morphology of various crystal phases and proportions thereof; and the diameter, the geometric structure, and the form of aggregation of secondary particles of the catalytically active component formed in the compounding process or the drying process. These factors are also closely related to the performance of the catalyst. That is, the object of the present invention may be considered to be related more particularly to a demand for a catalyst which simultaneously satisfies suppression of the production of a coke-like material, a high yield of the conjugated diolefin, and high mechanical strength in a reaction for producing a conjugated diolefin from a monoolefin having 4 or more carbon atoms.

The attrition resistance, which is an index representing the mechanical strength according to the present invention, is calculated by the following method. A tester for the attrition resistance of tablets manufactured by Hayashi Rikagaku K.K. is used as an apparatus, and 50 g of a catalyst sample is treated at a speed of rotation of 25 rpm for a treatment time of 10 minutes. Subsequently, the portion that has worn out is sieved through a standard sieve having a mesh size of 1.70 mm, and the weight of the catalyst remaining on the sieve is measured. The attrition resistance is calculated by the following formula. As the value of the attrition resistance is smaller, superior mechanical strength is obtained. A preferred range of the attrition resistance is, for example, 3% by weight or less, more preferably 1.5% by weight or less, and even more preferably 0.5% by weight or less.

```
Attrition resistance (wt%) = 100 × [(Weight of
catalyst - weight of catalyst remaining on sieve)/weight of
catalyst]
```

The conditions for the reaction of producing a conjugated diolefin from a monoolefin having 4 or more carbon atoms by means of the catalyst of the present invention include the following: when a mixed gas including from 1% by volume to 20% by volume of a monoolefin, from 5% by volume to 20% by volume of molecular oxygen, from 0% by volume to 60% by volume of water vapor, and from 0% by volume to 94% by volume of an inert gas, for example, nitrogen or carbon dioxide, is used as the mixed gas composition, the reaction bath temperature is in the range of from 200°C to 500°C, and the reaction pressure is from normal pressure to a pressure of 10 atmospheres, the space velocity (GHSV) of the mixed gas for the catalyst molded article of the present invention is in the range of from 350 hr$^{-1}$ to 7,000 hr$^{-1}$. Regarding the form of the reaction, the reaction may be performed in a fixed bed, a movable bed, and a fluidized bed, without any restrictions. However, a fixed bed is preferred.

The coke-like material according to the present invention is produced by at least any one of the reaction raw materials, the target product, and the reaction by-products in a reaction for producing a conjugated diolefin, and the details of the chemical composition or the production mechanism are not clearly understood. However, in the present invention, the coke-like material is regarded as a causative material that causes various problems, particularly such as inhibition of the circulation of reaction gases in an industrial plant, blocking of reaction tubes, and shutdown of the reaction resulting therefrom, when the coke-like material is precipitated in or adheres to the catalyst surface, inert materials, the interior of the reaction tube, or the interior of the facilities in the subsequent processes. Furthermore, for the purpose of avoiding the problems described above, in industrial plants, reactions are generally stopped before blocking occurs, and a regeneration treatment of combusting and removing coke-like materials by raising the reaction bath temperature, or the like. Regarding the mechanism for the production of coke-like materials, for example, the following is assumed. That is, examples may include that at the time of using a composite metal oxide catalyst including molybdenum, a coke-like material is produced by polymerization of various olefins and condensation of high-boiling point compounds, starting from the molybdenum compounds that have sublimed and precipitated inside the reactor; that the coke-like material is produced by polymerization of various olefins and condensation of high-boiling point compounds, starting from the abnormal acid-base points and radical production points inside the catalyst and the reactor; and that the coke-like material is produced by production of high-boiling point compounds as a result of the Diels-Alder reaction of the conjugated diene and other olefin compounds, and by condensation at sites where the temperature is locally low in the reactor. In addition to these, various mechanisms are known as well.

According to the present invention, a catalyst that may suppress the production of a coke-like material and may improve the long-term stability of the reaction, in regard to a reaction for producing a conjugated diolefin from a mixed gas including a monoolefin having 4 or more carbon atoms and molecular oxygen by catalytic oxidative dehydrogenation; and a method for producing the catalyst, may be obtained.

EXAMPLES

Hereinafter, the present invention will be described in more detail by way of Examples; however, the present invention is not intended to be limited to the following Examples as long as the gist is maintained. In the following description, unless particularly stated otherwise, percentage (%) means mol%. Also, in the following description, the definitions of the n-butene conversion ratio, the butadiene yield, and TOS are as follows.

```
n-Butene conversion ratio (mol%)
= (Number of moles of reacted n-butene/number of moles
of supplied n-butene) × 100
```

```
Butadiene yield (mol%)

= (Number of moles of produced butadiene/number of

moles of supplied n-butene) × 100

TOS = Mixed gas circulation time (hours)
```

X-ray diffraction peaks of the catalyst samples in Examples and Comparative Examples were measured as follows.

X-ray crystal structure analyzer: ULTIMA IV manufactured by Rigaku Corporation
Light source: Cu-K$\alpha$ radiation
Tube voltage: 40 kV
Tube current: 30 mA
Incident slit and light reception slit: Open
Scan speed: 10°/min
Sampling width: 0.02°

In a case in which the catalyst sample was not a powder but a molded article, the X-ray peaks were analyzed after the sample was pulverized and mixed for 10 minutes or longer using an agate mortar.

Example 1

(Production of Catalyst 1)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the solution was added to Mother Liquor 1. Subsequently, 165 parts by weight of bismuth nitrate was dissolved in an aqueous solution of nitric acid prepared by adding 42 parts by weight of nitric acid (60% by weight) to 175 ml of pure water that had been heated to 60°C, and the resulting solution was added to Mother Liquor 1. This Mother Liquor 1 was dried by a spray drying method, and a dried powder thus obtained was preliminarily calcined under the conditions of a calcination temperature of 440°C and a calcination time of 5 hours. Crystalline cellulose at a portion equivalent to 5% by weight was added to the preliminarily calcined powder thus obtained, the mixture was sufficiently mixed, and then the mixture was supported on an inert spherical support (silica-alumina) such that the support ratio would be 50% by weight, and was molded into a spherical shape, by a tumbling granulation method using a 33% by weight glycerin solution as a binder. The spherical molded article thus obtained was subjected to main calcination under the conditions of a calcination temperature of 505°C, a time for temperature increase of 5 hours, and a calcination time of 5 hours, and thereby Catalyst 1 of the present invention was obtained. The atomic ratio of Catalyst 1 calculated from the supplied raw materials was Mo : Bi : Fe : Co : Ni : Cs = 12 : 0.9 : 1.8 : 7.0 : 1.0 : 0.15, and the attrition resistance was 0.17% by weight. Furthermore, the relative intensity ratio of the X-ray diffraction peaks of Catalyst 1 of the present invention was Pr = 1.46. The results are presented in Table 1.

Example 2

(Production of Catalyst 2)

Production was carried out all in the same manner as in Example 1, except that the calcination temperature of the main calcination was changed to 520°C. The attrition resistance of Catalyst 2 of the present invention was 0.12% by weight, and the relative intensity ratio of the X-ray diffraction peaks was Pr = 0.97. The results are presented in Table 1.

Example 3

(Production of Catalyst 3)

Production was carried out all in the same manner as in Example 1, except that the time for temperature increase of the main calcination was changed to 9 hours, and the calcination temperature was changed to 500°C. The attrition resistance of Catalyst 3 of the present invention was 0.11% by weight, and the relative intensity ratio of the X-ray diffraction peaks was Pr = 1.97. The results are presented in Table 1.

Example 4

(Production of Catalyst 4)

Production was carried out all in the same manner as in Example 3, except that compounding was performed by changing the amount of addition of bismuth nitrate to 55 parts by weight, and changing the amount of nitric acid (60% by weight) to 15 parts by weight in 58 ml of pure water. The atomic ratio of Catalyst 4 calculated from the supplied raw materials was Mo : Bi : Fe : Co : Ni : Cs = 12 : 0.3 : 1.8 : 7.0 : 1.0 : 0.15, and the attrition resistance was 0.28% by weight. The relative intensity ratio of the X-ray diffraction peaks of Catalyst 4 of the present invention was Pr = 2.60. The results are presented in Table 1.

Example 5

(Production of Catalyst 5)

Production was carried out all in the same manner as in Example 3, except that compounding was performed by changing the amount of addition of bismuth nitrate to 92 parts by weight, and changing the amount of nitric acid (60% by weight) to 24 parts by weight in 97 ml of pure water. The atomic ratio of Catalyst 5 calculated from the supplied raw materials was Mo : Bi : Fe : Co : Ni : Cs = 12 : 0.5 : 1.8 : 7.0 : 1.0 : 0.15, and the attrition resistance was 0.66% by weight. The relative intensity ratio of the X-ray diffraction peaks of Catalyst 5 of the present invention was Pr = 2.71. The results are presented in Table 1.

Comparative Example 1

(Production of Catalyst 6)

Production was carried out all in the same manner as in Example 3, except that compounding was performed by changing the amount of addition of bismuth nitrate to 6 parts by weight, and changing the amount of nitric acid (60% by weight) to 2 parts by weight in 6 ml of pure water. The atomic ratio of Catalyst 6 calculated from the supplied raw materials was Mo : Bi : Fe : Co : Ni : Cs = 12 : 0.03 : 1.8 : 7.0 : 1.0 : 0.15, and the attrition resistance was 9.92% by weight. The relative intensity ratio of the X-ray diffraction peaks of Catalyst 6 for comparison was Pr = 3.84. The results are presented in Table 1.

Comparative Example 2

(Production of Catalyst 7)

800 parts by weight of ammonium heptamolybdate was completely dissolved in 3,000 parts by weight of pure water that had been heated to 80°C (Mother Liquor 1). Next, 6 parts by weight of cesium nitrate was dissolved in 62 ml of pure water, and the solution was added to Mother Liquor 1. Next, 275 parts by weight of ferric nitrate and 659 parts by weight of cobalt nitrate were dissolved in 495 ml of pure water that had been heated to 60°C, and the solution was added to Mother Liquor 1. Subsequently, 458 parts by weight of bismuth nitrate was dissolved in an aqueous solution of nitric acid prepared by adding 117 parts by weight of nitric acid (60% by weight) to 486 ml of pure water that had been heated to 60°C, and the solution was added to Mother Liquor 1. Subsequently, 28 parts by weight of calcium hydroxide was dispersed in 100 ml of pure water, and the dispersion was added to Mother Liquor 1. Subsequently, 97 parts by weight of magnesium nitrate was dissolved in 100 ml of pure water, and the solution was added to Mother Liquor 1. This Mother Liquor 1 was dried by a spray drying method, and a dried powder thus obtained was preliminarily calcined under the conditions of a calcination temperature of 440°C and a calcination time of 5 hours. Crystalline cellulose at a portion of 5% by weight was added to the preliminarily calcined powder obtained as described above, the mixture was sufficiently mixed, and then the mixture was supported on an inert spherical support (silica-alumina) such that the support ratio would be 50% by weight, and was molded into a spherical shape, by a tumbling granulation method using a 33% by weight glycerin solution as a binder. The spherical molded article thus obtained was subjected to main calcination under the conditions of a calcination temperature of 540°C, a time for temperature increase of 9 hours, and a calcination time of 5 hours, and thereby Comparative Catalyst 7 was obtained. The atomic ratio of Catalyst 7 calculated from the supplied raw materials was Mo : Bi : Fe : Co : Ni : Cs : Ca : Mg = 12 : 2.5 : 1.8 : 6.0 : 0 : 0.08 : 1.0 : 1.0, and the attrition resistance was 0.98% by weight. Furthermore, the relative intensity ratio of the X-ray diffraction peaks of Catalyst 7 for comparison was Pr = 0.76. The results are presented in Table 1.

[Table 1]

|  | Catalyst | Pi1 | Pi2 | Pr | Time for temperature increase in main calcination [hr] | Calcination temperature for main calcination [°C] |
|---|---|---|---|---|---|---|
| Example 1 | Catalyst 1 | 26122 | 17864 | 1.46 | 5.00 | 505 |
| Example 2 | Catalyst 2 | 23180 | 24000 | 0.97 | 5.00 | 520 |
| Example 3 | Catalyst 3 | 28134 | 14257 | 1.97 | 9.00 | 500 |
| Example 4 | Catalyst 4 | 47680 | 18316 | 2.60 | 9.00 | 500 |
| Example 5 | Catalyst 5 | 48184 | 17774 | 2.71 | 9.00 | 500 |
| Comparative Example 1 | Catalyst 6 | 54620 | 14233 | 3.84 | 9.00 | 500 |
| Comparative Example 2 | Catalyst 7 | 36279 | 47986 | 0.76 | 9.00 | 540 |

Test Example

(Coke precipitation reaction)

Catalysts including from Catalyst 1 of the present invention to Catalyst 7 for comparison thus obtained were reacted and evaluated by the following method. 53 ml of the catalyst was packed in a stainless steel reaction tube, and the catalyst was caused to react for a TOS of 310 hours using a mixed gas having a gas volume ratio of 1-butene : oxygen : nitrogen : water vapor = 1 : 1 : 7 : 1, under the conditions of normal pressure and a GHSV of 1,200 hr$^{-1}$, by changing the reaction bath temperature so as to maintain the 1-butene conversion ratio = 98.0 $\pm$ 2.0%. Thus, a coke-like material was precipitated on the catalyst. A liquid component and a gas component were separated at the reaction tube outlet using a condenser, and the components were respectively quantitatively analyzed by gas chromatography equipped with a hydrogen flame ionization detector and a thermal conductivity detector. The various data obtained by gas chromatography were compensated by multiplying by factor, and thus the 1-butene conversion ratio and the butadiene yield were calculated.

(Coke combustion reaction)

After the coke precipitation reaction, for the purpose of combusting the coke-like material precipitated on the catalyst, a combustion reaction was performed for about a TOS of 10 hours at a reaction bath temperature of 400°C using a mixed gas having a gas volume ratio of oxygen : nitrogen = 1 : 3 at normal pressure and at a space velocity of 400 hr$^{-1}$. A quantitative analysis similar to that performed for the coke precipitation reaction was performed, and it was considered that combustion of the coke-like material was completed at a time point at which the amounts of production of $CO_2$ and CO in the reaction tube outlet gas became zero. The amount of the coke-like material was calculated from the cumulative amounts of production of $CO_2$ and CO during the coke combustion reaction, as a percentage with respect to the weight of the catalyst packed therein.
The results for the coke precipitation reactions and the coke combustion reactions as described above are presented in Table 2. As is obvious from Table 2, it is understood that with regard to the catalyst of the present invention, when the relative intensity ratio of the X-ray diffraction peaks is within the range defined in the present invention, the production of the coke-like material may be suppressed to a large extent.

[Table 2]

|  | Catalyst | Pr | Amount of coke-like material adhered [wt%] | Butadiene yield in TOS of 280 hours [ %] |
|---|---|---|---|---|
| Test Example 1 | Catalyst 1 | 1.46 | 0.18 | 85.90 |
| Test Example 2 | Catalyst 2 | 0.97 | 0.06 | 86.60 |
| Test Example 3 | Catalyst 3 | 1.97 | 0.16 | 86.30 |
| Test Example 4 | Catalyst 4 | 2.60 | 0.14 | 87.71 |
| Test Example 5 | Catalyst 5 | 2.96 | 0.26 | 85.90 |
| Comparative Test Example 1 | Catalyst 6 | 3.86 | 1.77 | - |
| Comparative Test Example 2 | Catalyst 7 | 0.76 | 1.79 | 84.90 |

**Claims**

1. A composite metal oxide catalyst for conjugated diolefin production, used for producing a conjugated diolefin from a mixed gas including a monoolefin having 4 or more carbon atoms and molecular oxygen by a catalytic oxidative dehydrogenation reaction, the catalyst having a relative intensity ratio of X-ray diffraction peaks represented by the following Formula (A):

$$0.9 < Pr < 3.0 \qquad (A)$$

$$Pr = Pi1/Pi2$$

(in the formula, Pi1 represents the maximum peak height at a $2\theta$ value in the range of $26.4° \pm 0.3°$ in the X-ray diffraction peaks; Pi2 represents the maximum peak height at a $2\theta$ value in the range of $28.5° \pm 0.3°$ in the X-ray diffraction peaks; and Pr represents the relative intensity ratio of Pi1 with respect to Pi2).

2. The composite metal oxide catalyst for conjugated diolefin production according to claim 1, wherein the composite metal oxide satisfies the following compositional formula (D):

$$Mo_{12}Bi_aFe_bCo_cNi_dX_eY_fZ_gO_h \dots \qquad (D)$$

(in the formula, X represents at least one element of alkali metal selected from lithium, sodium, potassium, rubidium, and cesium; Y represents at least one element of alkaline earth metal selected from magnesium, calcium, strontium, and barium; Z represents at least one element selected from lanthanum, cerium, praseodymium, neodymium, samarium, europium, antimony, tungsten, lead, zinc, and thallium; a, b, c, d, e, f, and g respectively represent the atomic ratio of each component with respect to molybdenum 12, and are in the following ranges: $0.2 \leq a \leq 2.0$, $0.6 < b < 3.4$, $5.0 < c < 8.0$, $0 < d < 3.0$, $0 < e < 0.5$, $0 \leq f \leq 4.0$, and $0 \leq g \leq 2.0$; and h represents a value satisfying the oxidation state of the other elements).

3. A supported catalyst for conjugated diolefin production, the supported catalyst comprising the composite metal oxide catalyst according to claim 1 or 2 supported on a support.

4. The supported catalyst for conjugated diolefin production according to claim 3, wherein the average particle size is from 3.0 mm to 10.0 mm, and the support ratio of the composite metal oxide catalyst is from 20% by weight to 80 by weight.

5. A method for producing the catalyst for conjugated diolefin production according to any one of claims 1 to 4, the method comprising:

preparing a solution or a slurry including compounds containing the various metals of the composite metal oxide;
drying the solution or slurry to obtain a dried powder;
preliminarily calcining the dried powder at a temperature of from 200°C to 600°C;
molding the preliminarily calcined powder; and
subjecting the molded article to main calcination at a temperature of from 300°C to 600°C.

6. The method for producing the catalyst for conjugated diolefin production according to claim 5, wherein the solution or slurry is a solution or slurry including a compound containing molybdenum; a compound containing bismuth; a compound containing iron; a compound containing cobalt; a compound containing nickel; a compound containing at least one element of alkali metal selected from lithium, sodium, potassium, rubidium, and cesium; a compound containing at least one element of alkaline earth metal selected from magnesium, calcium, strontium, and barium; and a compound containing at least one element Z selected from lanthanum, cerium, praseodymium, neodymium, samarium, europium, antimony, tungsten, lead, zinc, and thallium.

7. A method for producing the supported catalyst for conjugated diolefin production according to claim 3 or 4, wherein the molding is coating a support with the composite metal oxide together with a binder.

[Figure 1]

**INTERNATIONAL SEARCH REPORT**

International application No.

PCT/JP2016/080838

A. CLASSIFICATION OF SUBJECT MATTER

*B01J23/887*(2006.01)i, *B01J35/08*(2006.01)i, *B01J37/08*(2006.01)i, *C07B61/00*
(2006.01)n, *C07C5/48*(2006.01)n, *C07C11/167*(2006.01)n

According to International Patent Classification (IPC) or to both national classification and IPC

B. FIELDS SEARCHED

Minimum documentation searched (classification system followed by classification symbols)
B01J23/887, B01J35/08, B01J37/08, C07B61/00, C07C5/48, C07C11/167

Documentation searched other than minimum documentation to the extent that such documents are included in the fields searched
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2013-202459 A (Mitsubishi Chemical Corp.), 07 October 2013 (07.10.2013), paragraphs [0013], [0051] to [0062]; fig. 1 (Family: none) | 1-7 |
| A | WO 2014/198901 A1 (BASF SE), 18 December 2014 (18.12.2014), claims 1 to 40; fig. 1, 2, 5 & JP 2016-521719 A claims 1 to 40; fig. 1, 2, 5 & US 2016/0145171 A1 & EP 3007823 A1 | 1-7 |

| ☒ | Further documents are listed in the continuation of Box C. | ☐ | See patent family annex. |

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 December 2016 (27.12.16) | 10 January 2017 (10.01.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

**INTERNATIONAL SEARCH REPORT**

International application No.

PCT/JP2016/080838

C (Continuation).    DOCUMENTS CONSIDERED TO BE RELEVANT

| Category* | Citation of document, with indication, where appropriate, of the relevant passages | Relevant to claim No. |
|---|---|---|
| A | WO 2014/051090 A1  (Asahi Kasei Chemicals Corp.), 03 April 2014 (03.04.2014), claims 1 to 13; fig. 4 to 16 & US 2015/0238939 A1 claims 1 to 13; fig. 4 to 16 & EP 2902106 A1 | 1-7 |
| A | JP 2013-043125 A  (Asahi Kasei Chemicals Corp.), 04 March 2013 (04.03.2013), claims 1 to 12; fig. 1 (Family: none) | 1-7 |
| A | JP 2014-073462 A  (Cosmo Oil Co., Ltd.), 24 April 2014 (24.04.2014), claims 1 to 4; fig. 1 to 4 (Family: none) | 1-7 |
| A | JP 2014-069128 A  (Nippon Shokubai Co., Ltd.), 21 April 2014 (21.04.2014), claims 1 to 4 (Family: none) | 1-7 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013100244 A
- JP 2011148765 A
- JP 2013146655 A
- JP 2013202459 A

**Non-patent literature cited in the description**

- **S.R.G. CARRAZFIN et al.** *App. Cat. A.,* 1996, vol. 135, 95-123
- **J.C. JUNG et al.** *Cat. Com.,* 2008, vol. 9, 2059-2062